# EUROPEAN PATENT SPECIFICATION

(11) **EP 3 434 782 B1**
(45) Date of publication and mention of the grant of the patent: **25.03.2020**
(21) Application number: 17779129.0
(22) Date of filing: 04.04.2017
(51) Int. Cl.: C12P 21/00, C12N 5/02, C12N 11/10, C12N 11/12, C12P 21/02, C12N 5/00, C12N 5/0735, C12N 5/074

(54) **PROTEIN PRODUCTION METHOD**
PROTEINHERSTELLUNGSVERFAHREN
PROCÉDÉ DE PRODUCTION DE PROTÉINES

(30) Priority: 04.04.2016 JP 2016075251
(43) Date of publication of application: 30.01.2019
(73) Proprietor: Nissan Chemical Corporation, Tokyo 103-6119 (JP); National Institute of Technology, Hachioji-shi, Tokyo 193-0834 (JP)
(72) Inventor: KANAKI, Tatsuro, Shiraoka-shi Saitama 349-0294 (JP); HAYASHI, Hisato, Funabashi-shi Chiba 274-0052 (JP); KAWAHARA, Hiroharu, Kitakyushu-shi Fukuoka 802-0985 (JP)
(74) Representative: J A Kemp LLP
(86) International application number: PCT/JP2017/014064
(87) International publication number: WO 2017/175751

(56) References cited:
- WO-A1-2015/111686
- WO-A1-2015/111686
- JP-A- S62 500 001
- JP-A- S62 500 001
- JP-A- 2004 141 301
- JP-A- 2011 107 722
- JP-A- 2013 066 745
- JP-A- 2013 067 075
- JUNG-YOUN SHIN ET AL: "Efficient formation of cell spheroids using polymer nanofibers", BIOTECHNOLOGY LETTERS, vol. 34, no. 5, 30 December 2011 (2011-12-30), pages 795-803, XP035053502, ISSN: 1573-6776, DOI: 10.1007/S10529-011-0836-9

### SEQUENCE LISTING

<110> NISSAN CHEMICAL INDUSTRIES, LTD.
   National Institute of Technology, Japan
<120> Method for producing protein
<130> 092594
<150> JP 2016-075251
   <151> 2016-04-04
<160> 2
<170> Patent In version 3.5
<210> 1
   <211> 20
   <212> DNA
   <213> Artificial Sequence
<220>
   <223> Forward Primer
<400> 1
   atgaccaaca agtgtctcct 20
<210> 2
   <211> 20
   <212> DNA
   <213> Artificial Sequence
<220>
   <223> Reverse Primer
<400> 2
   tcagtttcgg aggtaacctg 20

## Description

### [Technical Field]

The present invention relates to a technique for producing a protein by cell culture.

### [Background Art]

In recent years, in the field of life science, it has become very important to produce useful proteins (antibody, vaccine etc.) of interest on an industrial scale by large-scale culture of animal cells that produce the desired useful proteins. In the production of useful proteins by using animal cell culture, CHO-DG44 cells and the like capable of suspension culture are used to give antibodies and the like, and MDCK cells and Vero cells adhered to microcarrier particles are cultured to give vaccines and the like. However, when proteins are produced by CHO-DG44 culture, it needs to be performed under the constraints of many already established rights. Furthermore, while a human glycosylated antibody production method using human cells has recently been developed, PerC6 cells under development similarly needs to be performed under the constraints of many rights. HEK 293 cells also derived from human is not suitable for suspension culture because it is adhesive. Furthermore, cells having the ability to suspend and proliferate are associated with a risk of canceration. Thus, cells to be the post CHO-DG44 cells do not appear.

The microcarrier method used for vaccine production is a method for culturing MDCK cells and Vero cells while adhering them to the surface of a carrier. This method requires stirring culture. However, the stirring poses a problem of cell damage on the surface due to the collision of the carriers. This cell damage exerts an adverse effect on the efficiency of protein production, and therefore, establishment of a new suspension culture method to replace the microcarrier method has been desired.

Relating to cell culture, the present inventors particularly reported a protein production accelerating agent suitable for high production of protein by animal cells and a medium containing the same, a protein production method using the protein production accelerating agent and a medium containing the same (patent document 1).

In addition, it has been reported that suspension culture of animal and plant cells and/or tissues can be performed while keeping them still by mixing a nanofiber composed of polysaccharides such as cellulose, chitin and the like in a liquid medium, without substantially increasing the viscosity of the liquid medium, and that the proliferation activity of the cell is promoted by culture using this medium composition (patent document 2).

### [Document List]

### [Patent documents]

patent document 1: WO 2014/030726 A1
patent document 2: WO 2015/111686 A1

### [Summary of Invention]

### [Technical Problem]

The problem of the present invention is to provide a technique for efficiently producing a protein by suspension culture of adherent cells.

### [Solution to Problem]

The present inventors have conducted intensive studies and found that the amount of protein production remarkably increases by culturing protein-producing cells selected from HEK293, CHO-K1, BHK-21, MDCK, Vero, HepG2, MCF-7 in suspension in a liquid medium containing nanofibers composed of polysaccharides selected from cellulose, chitin and chitosan under stirring condition and in a state of being attached to the nanofiber, which resulted in the completion of the present invention.

That is, the present invention provides the following:
[1] A method for producing a protein, comprising culturing a cell having protein production ability in suspension in a medium composition comprising nanofibers under physical disruption condition and in a state of being attached to the nanofiber,
   wherein the nanofiber is constituted of a non-water-soluble polysaccharide selected from the group consisting of cellulose, chitin and chitosan
   the cell is HEK293, CHO-K1, BHK-21, MDCK, Vero, HepG2 or MCF-7, and the nanofibers in the medium composition are present at a concentration within a range that can promote the protein production and/or proliferation by the cell to be cultured.
[2] The method of [1], wherein the nanofibers are chitin nanofibers.
[3] The method of [2], wherein the content of the chitin nanofibers in the medium composition is 0.003 - 0.1% (weight/volume).
[4] The method of any of [1] to [3], wherein the cell is an adherent cell.

### [Advantageous Effects of Invention]

According to the present invention, cells can be efficiently proliferated, and large-scale production of proteins such as enzyme, cell growth factor, antibody and the like can be performed.

### [Description of Embodiments]

The present invention is explained in more detail in the following.

The terms used in the present specification are defined as follows.

The cell in the present invention is HEK293 (human embryonic kidney cells), HepG2 (human liver cancer cell line), MDCK, CHO-K1, BHK-21, Vero, HepG2 or MCF-7.

The tissue is a unit of a structure which is an assembly in a certain manner of cells having some kinds of different properties and functions.

When cells and/or tissues are cultivated, the cells and/or tissues to be cultivated can be selected freely from the cells and/or tissues described above and cultivated. The cells and/or tissues can be directly recovered from an animal or plant body. The cells and/or tissues may be induced, grown or transformed from an animal or plant body by applying a particular treatment and then collected. In this case, the treatment may be in vivo or in vitro.

Suspending of cells in the present invention refers to a state where cells do not adhere to a culture container (non-adhesive). Furthermore, in the present invention, when the cells are proliferated, differentiated or maintained, the state where the cells are uniformly dispersed and suspended in the liquid medium composition in the absence of a pressure on or vibration of the liquid medium composition from the outside or shaking, rotating operation and the like in the composition is referred to as "standing in suspension", and cultivation of the cells in such condition is referred to as "static suspension culture". In the "standing in suspension", the period of suspending includes not less than at least 5 min, preferably not less than 1 hr, not less than 24 hr, not less than 48 hr, not less than 6 days, not less than 21 days, though the period is not limited thereto as long as the suspended state is maintained.

### Nanofibers

The nanofibers contained in the medium composition to be used in the present invention show an effect of suspending cells in a liquid medium. For example, nanofibers obtained by refining a comparatively large fiber structure composed of a polymer compound by a high-pressure treatment can be recited as the nanofibers contained in the medium composition to be used for the method of the present invention.

In the present specification, nanofiber refers to a fiber having an average fiber diameter (D) of 0.001 to 1.00 µm. The average fiber diameter of the nanofiber to be used in the present invention is preferably 0.005 to 0.50 µm, more preferably 0.01 to 0.05 µm, further preferably 0.01 to 0.02 µm. When the average fiber diameter is less than 0.001 µm, the nanofiber may be too fine to achieve the suspending effect, which in turn may prevent improvement of the property of the medium composition containing the same.

The aspect ratio (L/D) of the nanofiber to be used in the present invention is obtained from average fiber length/average fiber diameter and is generally 2 - 500, preferably 5 - 300, more preferably 10 - 250. When the aspect ratio is less than 2, the nanofiber lacks dispersibility in the medium composition and the suspending action may not be obtained sufficiently. When it exceeds 500, the fiber length becomes extremely large and the viscosity of the composition increases, which in turn may hinder passage operations such as medium change and the like. In addition, since the medium composition does not easily transmit visible light, transparency becomes low, time-course observation of cultured cells becomes difficult, and cell evaluation using absorbance, fluorescence, luminescence, and the like may be prevented.

In the present specification, the average fiber diameter (D) of the nanofiber is determined as follows. First, a collodion support film manufactured by Okenshoji Co., Ltd. is subjected to a hydrophilic treatment for 3 min by an ion cleaner (JIC-410) manufactured by JEOL Ltd., several drops of an evaluation target nanofiber dispersion solution (diluted with ultrapure water) is added dropwise, and dried at room temperature. The film is observed by a transmission electron microscopy (TEM, H-8000) (x10,000) manufactured by Hitachi, Ltd. at acceleration voltage 200 kV, the fiber diameter of each of the specimen number: 200 - 250 nanofibers is measured using the obtained images, and the number average value thereof is taken as the average fiber diameter (D).

The average fiber length (L) is determined as follows. The evaluation target nanofiber dispersion solution is diluted with pure water to 100 ppm, and nanofibers are uniformly dispersed by an ultrasonic cleaning machine. The nanofiber dispersion solution is cast on a silicon wafer with a surface hydrophilized in advance with concentrated sulfuric acid and dried at 110°C for 1 hr to give a sample. Using the images of the obtained sample observed by a scanning electron microscope (SEM, JSM-7400F) (x2,000) manufactured by JEOL Ltd., the fiber length of each of the specimen number: 150 - 250 nanofibers is measured, and the number average value thereof is taken as the average fiber length (L).

When mixed with the liquid medium, the nanofibers to be used in the present invention are uniformly dispersed in the liquid while maintaining the primary fiber diameter, and cells attach to the dispersed nanofibers, thus preventing sedimentation of the cells.

The starting materials constituting the nanofiber are non-water-soluble polysaccharides selected from cellulose, chitin, and chitosan.

Cellulose is a natural polymer compound in which D-glucopyranose, which is a 6-membered ring of glucose, is bonded with β-1,4 glucoside. As the starting materials, plant-derived cellulose such as lumber, bamboo, hemp, jute, kenaf, cotton, agricultural crop/food residues and the like, or cellulose produced by microorganism or animal such as bacteria cellulose, Cladophorales (Cladophora), gray plant (Glaucophyceae), Valonia, sea squirt cellulose and the like can be used. The plant-derived cellulose has a very thin fiber called microfibril and the microfibrils are further bundled and stepwisely form higher order structures of fibril, lamella and fiber cell. In bacterial cellulose, moreover, microfibrils of cellulose secreted from bacterial cells form a fine network structure while maintaining the thickness thereof.

In the present invention, highly pure cellulose starting materials such as cotton, bacteria cellulose and the like can be used as they are. Other plant-derived cellulose and the like are preferably used after isolation and purification. In the present invention, cotton cellulose, bacteria cellulose, kraft pulp cellulose, microcrystalline cellulose and the like are preferably used. Particularly, kraft pulp cellulose is preferably used since it has a high suspending action.

The chitinous substance refers to one or more carbohydrates selected from the group consisting of chitin and chitosan. The main sugar units constituting chitin and chitosan are N-acetylglucosamine and glucosamine, respectively. In general, those having a high content of N-acetylglucosamine and poorly soluble in an acidic aqueous solution are chitin, and those having a high content of glucosamine and soluble in acidic aqueous solution are chitosan. In the present specification, those having a proportion of N-acetylglucosamine in the constituent sugar of not less than 50% are called chitin, and those having a proportion of less than 50% are called chitosan for convenience. To achieve a high suspension action, a higher proportion of N-acetylglucosamine in the sugar unit constituting chitin is more preferable. The proportion of N-acetylglucosamine in the sugar unit constituting chitin is preferably not less than 80%, more preferably not less than 90%, further preferably not less than 98%, most preferably 100%.

As the starting material of chitin, many biological resources such as shrimp, crab, insect, shellfish, mushroom and the like can be used. The chitin to be used in the present invention may be chitin having an α type crystal structure such as chitin derived from crab shell and shrimp shell, and the like or chitin having a β type crystal structure such as chitin derived from cuttlebone and the like. Crabs and shrimp outer shells are often treated as industrial waste and are preferred as starting materials from the viewpoint of easy availability and effective use. However, a deproteinization step and a deashing step are required for removal of proteins, ash content and the like contained as impurities. In the present invention, therefore, purified chitin that already underwent a matrix removal treatment is preferably used. Purified chitin is commercially available.

### Preparation of nanofibers

The medium composition to be used in the present invention contains nanofibers prepared from cellulose, chition or chitosan.

When the starting material of the nanofiber is cellulose, chitin or chitosan, the nanofibers are generally obtained by pulverizing the starting material. While the pulverization method is not limited, a method affording a strong shear force such as a medium stirring mill, for example, a high-pressure homogenizer, a grinder (stone mill), a bead mill, and the like are preferable for refining to the below-mentioned fiber diameter or fiber length that meets the object of the present invention.

Among these, refining using a high-pressure homogenizer is preferable. For example, refining (pulverization) using a wet pulverization method such as those disclosed in JP-A-2005-270891 and JP-B-5232976 is desirable. To be specific, the starting material is pulverized by injecting a dispersion of the raw material from each of a pair of nozzles at a high pressure to cause collision thereof. For example, Star Burst system (high-pressure pulverization apparatus manufactured by SUGINO MACHINE LIMITED) and NanoVater (high-pressure pulverization apparatus manufactured by yoshida kikai co., ltd.) are used therefor.

When the starting material is refined (pulverized) using the aforementioned high-pressure homogenizer, the degree of refinement and homogenization depends on the pressure pumped to the ultrahigh pressure chamber of the high pressure homogenizer, the number of passages through the ultrahigh pressure chamber (number of treatments), and the concentration of the starting material in the aqueous dispersion. The pumping pressure (treatment pressure) is generally 50 - 250 MPa, preferably 150 - 245 MPa. When the pumping pressure is less than 50 MPa, refining of the nanofiber may become insufficient, and the effects expected by refining may not be obtained.

The concentration of the starting material in the aqueous dispersion during the refining treatment is 0.1 mass % - 30 mass %, preferably 1 mass % - 10 mass %. When the concentration of the starting material in the aqueous dispersion is less than 0.1 mass %, the producibility is low, and when it is higher than 30 mass %, the pulverization efficiency is low and the desired nanofiber cannot be achieved. The number of the refining (pulverization) treatments is not particularly limited. Depending on the concentration of the starting material in the aforementioned aqueous dispersion, the number of treatments to achieve sufficient refining is about 10 - 100 when the concentration of the starting material is 0.1 - 1 mass %; however, it needs to be about 10 - 1000 when the concentration is 1 - 10 mass %. A high concentration exceeding 30 mass % is industrially unrealistic since the number of treatments needs to be not less than some thousands and the viscosity becomes high to the extent that handling is hindered.

The concentration of the nanofibers in the medium composition of the present invention can be appropriately set within a range that can promote the protein production and/or proliferation by the cell to be cultured.

In the case of chitin nanofiber, it can be added to the medium at a concentration of generally 0.003 - 0.1% (weight/volume), preferably 0.003 - 0.03% (weight/volume), more preferably 0.01 - 0.03% (weight/volume).

In the case of cellulose nanofiber, it can be added to the medium at a concentration of generally 0.0001% to 1.0% (weight/volume), for example, 0.0005% to 1.0% (weight/volume), preferably 0.001% to 0.5% (weight/volume), more preferably 0.01% to 0.1% (weight/volume), further preferably 0.01% to 0.05% (weight/volume).

In the case of pulp cellulose nanofiber among cellulose nanofibers, the lower limit of the concentration in the medium is preferably not less than 0.01% (weight/volume), not less than 0.015% (weight/volume), not less than 0.02% (weight/volume), not less than 0.025% (weight/volume), or not less than 0.03% (weight/volume). In the case of pulp cellulose nanofiber, the upper limit of the concentration in the medium is preferably not more than 0.1% (weight/volume) or not more than 0.04% (weight/volume).

In the case of microcrystalline cellulose nanofiber, the lower limit of the concentration in the medium is preferably not less than 0.01% (weight/volume), not less than 0.03% (weight/volume), or not less than 0.05% (weight/volume). In the case of microcrystalline cellulose nanofiber, the upper limit of the concentration in the medium is preferably not more than 0.1% (weight/volume).

Non-water-soluble nanofibers such as cellulose nanofibers, chitin nanofibers and chitosan nanofibers can be handled without substantially increasing the viscosity of the medium composition when the concentration thereof is generally not more than 0.1% (weight/volume).

### Medium

When the culture target cell is derived from an animal (particularly mammal), any medium generally used for culturing animal cells (preferably, mammalian cells) can be used. Examples of the medium include Dulbecco's Modified Eagle's Medium (DMEM), hamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), StemPro hESC SFM (manufactured by Invitrogen), mTeSR1 or 2 medium (manufactured by STEMCELL Technologies), Sf-900II (manufactured by Invitrogen), Opti-Pro (manufactured by Invitrogen), and the like.

When the culture target cell is derived from a plant, a medium obtained by adding auxins and, where necessary, a plant growth control substance (plant hormone) such as cytokines and the like at a suitable concentration to a basic medium such as Murashige Skoog (MS) medium, Linsmaier Skoog (LS) medium, White medium, Gamborg's B5 medium, niche medium, hela medium, Morel medium and the like generally used for culture of plant tissues, or a modified medium wherein these medium components are modified to an optimal concentration (e.g., ammonia nitrogen at a half concentration etc.) can be mentioned as the medium. These media can be further supplemented, where necessary, with casein degrading enzyme, corn steep liquor, vitamins and the like. Examples of the auxins include, but are not limited to, 3-indoleacetic acid (IAA), 3-indolebutyric acid (IBA), 1-naphthaleneacetic acid (NAA), 2,4-dichlorophenoxyacetic acid (2,4-D) and the like. For example, auxins can be added to a medium at a concentration of about 0.1 - about 10 ppm. Examples of the cytokines include, but are not limited to, kinetin, benzyladenine (BA), zeatin and the like. For example, cytokines can be added to a medium at a concentration of about 0.1 - about 10 ppm.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture of animal-derived cells and/or tissues, those of ordinary skill in the art can also add, according to the object, one or more kinds of other chemical components and biogenic substances in combination. Examples of the components to be added to a medium for animal-derived cells and/or tissues include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various proliferation factors, various extracellular matrices, various cell adhesion molecules and the like. Examples of the cytokine to be added to a medium include, but are not limited to, interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony stimulating factor (G-CSF), monocyte colony stimulating factor (M-CSF), granulocyte-macrophage colony stimulating factor (GM-CSF), stem cell factor (SCF), flk2/flt3 ligand (FL), leukemia cell inhibitory factor (LIF), oncostatin M (OM), erythropoietin (EPO), thrombopoietin (TPO) and the like.

Examples of the hormone to be added to a medium include, but are not limited to, melatonin, serotonin, thyroxine, triiodothyronine, epinephrine, norepinephrine, dopamine, anti-Mullerian hormone, adiponectin, adrenocorticotropic hormone, angiotensinogen and angiotensin, antidiuretic hormone, atrial natriuretic peptide, calcitonin, cholecystokinin, corticotropin release hormone, erythropoietin, follicle stimulating hormone, gastrin, ghrelin, glucagon, gonadotropin release hormone, growth hormone release hormone, human chorionic gonadotropin, human placental lactogen, growth hormone, inhibin, insulin, insulin-like growth factor, leptin, luteinizing hormone, melanocyte stimulating hormone, oxytocin, parathyroid hormone, prolactin, secretin, somatostatin, thrombopoietin, thyroid gland stimulation hormone, thyrotropin releasing hormone, cortisol, aldosterone, testosterone, dehydroepiandrosterone, androstenedione, dihydrotestosterone, estradiol, estrone, estriol, progesterone, calcitriol, calcidiol, prostaglandin, leukotriene, prostacyclin, thromboxane, prolactin releasing hormone, lipotropin, brain natriuretic peptide, neuropeptide Y, histamine, endothelin, pancreas polypeptide, rennin and enkephalin.

Examples of the growth factor to be added to a medium include, but are not limited to, transforming growth factor-α (TGF-α), transforming growth factor-β (TGF-β), macrophage inflammatory protein-la (MIP-1α), epithelial cell growth factor (EGF), fibroblast growth factor-1, 2, 3, 4, 5, 6, 7, 8 or 9 (FGF-1, 2, 3, 4, 5, 6, 7, 8, 9), nerve cell growth factor (NGF) hepatocyte growth factor (HGF), leukemia inhibitory factor (LIF), protease nexin I, protease nexin II, platelet-derived growth factor (PDGF), choline vasoactive differentiation factor (CDF), chemokine, Notch ligand (Delta1 and the like), Wnt protein, angiopoietin-like protein 2, 3, 5 or 7 (Angpt2, 3, 5, 7), insulin like growth factor (IGF), insulin-like growth factor binding protein (IGFBP), Pleiotrophin and the like.

In addition, these cytokines and growth factors having amino acid sequences artificially altered by gene recombinant techniques can also be added. Examples thereof include IL-6/soluble IL-6 receptor complex, Hyper IL-6 (fusion protein of IL-6 and soluble IL-6 receptor) and the like.

Examples of the various extracellular matrices and various cell adhesion molecules include collagen I to XIX, fibronectin, vitronectin, laminin-1 to 12, nitogen, tenascin, thrombospondin, von Willebrand factor, osteopontin, fibrinogen, various elastins, various proteoglycans, various cadherins, desmocolin, desmoglein, various integrins, E-selectin, P-selectin, L-selectin, immunity globulin superfamily, Matrigel, poly-D-lysine, poly-L-lysine, chitin, chitosan, sepharose, hyaluronic acid, alginate gel, various hydrogels, cleavage fragments thereof and the like.

Examples of the antibiotic to be added to a medium include sulfa preparations, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, piromidic acid, ciprofloxacin, cinoxacin, norfloxacin, perfloxacin, rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, adinoshirin and sulbactam formaldehyde hudrate ester, tazobactam, aztreonam, sulfazethin, isosulfazethin, norcardicin, m-carboxyphenyl, phenylacetamidophosphonic acid methyl, chlortetracycline, oxytetracycline, tetracycline, demeclocycline, doxycycline, methacycline, and minocycline.

### Production method of medium composition

The medium composition to be used in the present invention can be produced by mixing the above-mentioned nanofibers with a liquid medium for cell culture to a concentration capable of promoting protein production and/or proliferation by the culture target cells.

The shape of the nanofibers may be a formulated solid such as powder, tablet, pill, capsule, granule, or a liquid such as a dispersion in an appropriate physiological aqueous solvent, or may be bonded to a substrate or a single substance. Examples of the additive used for formulating include preservatives such as p-oxybenzoic acid esters and the like; excipients such as lactose, glucose, sucrose, mannit and the like; lubricants such as magnesium stearate, talc and the like; binders such as polyvinyl alcohol, hydroxypropylcellulose, gelatin and the like; surfactants such as fatty acid ester and the like; plasticizers such as glycerol and the like. These additives are not limited to those mentioned above, and can be selected freely as long as they are utilizable for those of ordinary skill in the art. The sterilization method is not particularly limited, and, for example, radiation sterilization, ethylene oxide gas sterilization, autoclave sterilization, filter sterilization and the like can be mentioned.

In a preferable embodiment, a medium composition to be used in the present invention is prepared by mixing a dispersion of the above-mentioned nanofibers in a physiological aqueous solvent and a liquid medium. The dispersion may be sterilized (autoclave, gamma ray sterilization etc.). Alternatively, the dispersion and a liquid medium (aqueous solution of the medium) prepared by dissolving a powder medium in water may be mixed, sterilized and used. Sterilization of the dispersion and the liquid medium may be carried out separately before mixing. Examples of the aqueous solvent include, but are not limited to, water, dimethyl sulfoxide (DMSO) and the like. As the aqueous solvent, water is preferable. The aqueous solvent may contain an appropriate buffering agent or salt. The above-mentioned nanofiber dispersion is useful as a medium additive for preparing the medium composition to be used in the present invention.

The mixing ratio of nanofiber dispersion:liquid medium (aqueous solution of medium) is generally 1:99 - 99:1, preferably 10:90 - 90:10, more preferably 20:80 - 80:20.

Examples of the preparation method of the medium composition containing nanofiber are shown below, which are not to be construed as limitative. The nanofibers are added to ion exchange water or ultrapure water. The mixture is stirred at room temperature until the whole is dispersed uniformly, and then sterilized (e.g., autoclave sterilization at 121°C for 20 min). The aforementioned sterilized nanofiber dispersion is added to a given medium to be used for static culture while stirring the medium (e.g., homomixer etc.) to uniformly mix the dispersion with the medium. The mixing method of the aqueous solution and the medium is not particularly limited, and may be manual mixing such as pipetting etc., or mixing with an instrument such as magnetic stirrer, mechanical stirrer, homomixer and homogenizer.

In one embodiment, a medium composition containing the aforementioned nanofibers is capable of standing cells in suspension at at least one point in the temperature range (e.g., 0 - 40°C) where cells can be maintained and cultured. The medium composition of the present invention is capable of standing cells in suspension at at least one point in the temperature range of preferably 25 - 37°C, most preferably 37°C. The medium composition can be produced by, for example, the method described in WO 2015/111686 A1.

### Culture method

The present invention provides a method for culturing cells in suspension in the above-mentioned medium composition under physical disruption condition and in a state of being attached to the nanofibers.

The nanofibers to be used in the present invention show an effect of suspending cells and/or tissues in a liquid containing the nanofibers (preferably an effect of standing the cells and/or tissues in suspension) when the cells and/or tissues are cultured ex vivo (WO 2015/111686 A1). Therefore, when the target cells are cultured in the above-mentioned medium composition, the cells attach to the nanofiber dispersed in the medium composition. Thus, it is possible to culture the cells while uniformly dispersing them in the liquid medium composition and maintaining the suspension state, even without physical disruption such as stirring, shaking operation and the like. Using the above-mentioned medium composition, cells can be cultured in suspension even without physical disruption such as stirring, shaking operation and the like, and cell proliferation is promoted thereby (WO 2015/111686 A1). In conventional suspension culture methods, it is considered that the proliferation rate and recovery rate of the cells are low or the functions of the cells are impaired because a shear force acts on the cells when stirring or shaking operation is involved. When cells are cultured in suspension in a medium composition containing the aforementioned nanofibers intentionally under physical disruption conditions, surprisingly, cell proliferation is remarkably promoted and production of protein is remarkably enhanced as compared to static suspension culture.

Examples of the physical disruption condition include, but are not limited to, stirring, shaking, rotating and the like. Stirring is performed by mixing cells and a medium with a stirring bar (stirrer) or a screw. Shaking is performed by shaking the culture container at a certain frequency to mix cells and a medium. Examples of preferable physical disruption conditions include shaking at 20 - 150 rpm, preferably 50 - 130 rpm, more preferably 80 - 120 rpm, further preferably 90 - 110 rpm (e.g., 100 rpm). The shaking can be performed by, for example, EYELA Multishaker MMS (Tokyo Rikakikai Co. Ltd.).

Using the culture method of the present invention, cells are efficiently proliferated. Thus, the culture method of the present invention is superior as a method for proliferating cells or a method for enhancing proliferation of cells. When cells are cultured by the method of the present invention, the cells do not adhere to the culture container but attach to the nanofibers dispersed in the medium composition, the cells are not localized only on the bottom surface of the culture container but dispersed with three-dimensional spread, and the cells are promoted to proliferate by the application of physical disruption. Particularly, when chitin nanofibers are used as the nanofibers, cells attach to the chitin nanofiber, strongly proliferate therefrom as a scaffold, and the proliferated cells are connected in the form of grape cells on the nanofiber. To achieve this proliferation promoting effect, nanofibers only need to be contained in the medium composition at a concentration sufficient for suspending cells and/or tissues (i.e., avoiding adhesion of cells and tissues to culture container), and capability of standing in suspension (i.e., that the cells and/or tissues are uniformly dispersed in a liquid medium composition and are in a suspended state without pressure, vibration, shaking, rotation operation and the like from the outside) is not essential. For example, in the case of chitin nanofiber, a proliferation enhancing effect is provided as long as the concentration is not less than 0.0001% (weight/volume), preferably, not less than 0.003% (weight/volume), sufficient for expressing a suspending action, even when the concentration is not more than 0.03% (weight/volume) (e.g., not more than 0.025% (weight/volume), not more than 0.02% (weight/volume)) that enables stable static suspension culture.

Among nanofibers, chitin nanofiber is particularly superior in the cell proliferation enhancing effect.

In the culture method of the present invention, any of non-adherent cell and adherent cell can be used selected from HEK293, CHO-K1, BHK-21, MDCK, Vero, HepG2 or MCF-7. An adherent cell is a cell that requires a scaffold for growth and proliferation. Non-adherent cell is a cell that does not require a scaffold for growth and proliferation. In the culture method of the present invention, adherent cell is preferably used. When adherent cells are used in the method of the present invention, the adherent cells do not adhere to the bottom surface of the culture container, are not localized only on the bottom surface of the culture container but dispersed with three-dimensional spread, and proliferate in a state of being attached to the nanofiber. Particularly, when chitin nanofibers are used as the nanofibers, the cells attach to the chitin nanofiber, strongly proliferate therefrom as a scaffold, and the proliferated cells are connected in the form of grape cells on the nanofiber. Thus, suspension culture of adherent cells becomes possible. As a result, proliferation of adherent cell is enhanced than when cultured in a state of being adhered to the bottom surface of the container. In addition, adherent cells can be cultured at high density than when cultured in a state of being adhered to the bottom surface of the container.

In the culture method of the present invention, suspension culture of adherent cells is performed. Thus, after suspension culture of adherent cells by the culture method of the present invention, the cells can be passaged by simply adding a fresh medium composition of the present invention to the culture product after culturing, or adding the whole or a part of the culture product after culturing to a fresh medium composition of the present invention, without requiring a cell detaching operation from the culture container. Using the passage culture method adherent cells can be passaged without a cell detaching operation from the culture container. In addition, using the passage culture method the culture scale of adherent cells can be enlarged without a cell detaching operation from the culture container. As a cell detaching operation from the culture container, a treatment with a chelating agent (e.g., EDTA) and/or protease (e.g., trypsin, collagenase) can be mentioned. The passage culture method is advantageous for passage culture of adherent cells having high sensitivity to a cell detaching operation from the culture container (e.g., adherent cell with lowered survivability by detaching operation, adherent cell with character susceptible to change by detaching operation). Examples of the adherent cells having high sensitivity to a cell detaching operation from the culture container are cells such as MDCK cell, HEK293 cell and CHO cell.

Using the culture method of the present invention, adherent cells can be cultured at a high density, the cells can be proliferated efficiently, and protein production efficiency can be increased. Therefore, the culture method of the present invention is useful for protein production by in vitro cell culture. Cells having the desired protein productivity are subjected to suspension culture under physical disruption conditions in a state of being attached to the nanofiber in a medium composition containing the aforementioned nanofibers, and the protein is isolated from the culture product, whereby the protein substance can be obtained. Examples of the protein include, but are not limited to, antibody, enzyme (urokinase etc.), hormone (insulin etc.), cytokine (interferon, interleukin, tumor necrosis factor, colony stimulating factor, growth factor etc.), vaccine antigen, and other physiologically active proteins. Examples of the cell producing the desired protein include HEK293, CHO-K1, BHK-21, MDCK, Vero, HepG2, MCF-7 and the like, into which gene encoding the protein of interest or gene involved in the biosynthesis of the protein has been introduced. Cells used for the production of recombinant proteins are well known to those skilled in the art and those cells can be used as appropriate in the methods of the present invention. To enlarge the culture scale, as mentioned above, a fresh medium composition containing the above-mentioned nanofibers may be added to the culture product after culturing, or the whole or a part of the culture product after culturing may be added to a fresh medium composition containing the nanofibers, without performing a cell detaching operation from the culture container. When the desired protein is isolated from the culture product, it is necessary to remove the cells from the culture product. In the method of the present invention, cells are suspended in the medium composition while being attached to nanofiber. Therefore, the cells can be removed by a convenient method such as centrifugation, filtration treatment and the like. In addition, the nanofibers in the medium composition can also be removed by a convenient method such as centrifugation, filtration treatment and the like. A method for isolating the protein from the culture product is well known to those of ordinary skill in the art and, for example, biochemical separation and purification methods of physiologically active substance such as chromatography (e.g., chromatographys such as ion exchange chromatography, hydrophobic chromatography, affinity chromatography, reversed-phase chromatography) are applicable.

When cells are cultivated by the culture method of the present invention, culture tools generally used for cell culture such as schale, flask, plastic bag, Teflon (registered trade mark) bag, dish, petri dish, tissue culture dish, multidish, microplate, microwell plate, multiplate, multiwall plate, chamber slide, tube, tray, culture bag, roller bottle and the like can be used for culturing. While the materials of these culture tools are not particularly limited, for example, glass, plastics such as polyvinyl chloride, cellulosic polymers, polystyrene, polymethylmethacrylate, polycarbonate, polysulfone, polyurethane, polyester, polyamide, polystyrene, polypropylene and the like, and the like can be mentioned. Moreover, various surface treatments (e.g., plasma treatment, corona treatment etc.) may be applied to these plastics. Furthermore, these culture tools may be coated in advance with an extracellular matrix, a cell adhesion molecule and the like.

The cells and/or tissues can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high density culture. As a method for supplying a new medium and feeding the required substances to the cells and/or tissues during the culture using such apparatuses, fed-batch culture, continuous culture and perfusion culture are available, and all these methods can be used for the culture method of the present invention.

The temperature when cells and/or tissues are cultivated is generally 25 to 39°C, preferably 33 to 39°C (e.g., 37°C), for animal cells. The CO₂ concentration is generally 4 to 10% by volume, preferably 4 to 6% by volume (e.g., 5% by volume), in the culture atmosphere. The culture period is generally 3 to 35 days, preferably 7 to 20 days, more preferably 10 to 14 days, which may be freely set according to the object of the culture. The culture temperature for plant cells is generally 20 to 30°C and, when light is necessary, they can be cultured under illuminance conditions of illuminance 2000 - 8000 lux. While the culture period is generally 3 to 70 days, it can be freely set according to the object of the culture. In the method of the present invention, suspension culture is performed throughout the whole culture period under physical disruption conditions.

When cells are cultivated by the method of the present invention, cells prepared separately are added to the culture composition and mixed to give a uniform dispersion. In this case, the mixing method is not particularly limited and, for example, manual mixing using pipetting and the like, mixing using instrument such as stirrer, vortex mixer, microplate mixer, shaking machine and the like can be mentioned. After mixing, suspension culture is performed under physical disruption conditions. When the medium composition needs to be exchanged during the culture period, the cells and the medium composition are separated by centrifugation or filtration treatment, and a medium composition containing fresh nanofiber can be added of the cells. Alternatively, the cells are appropriately concentrated by centrifugation or filtration treatment, and a medium composition containing fresh nanofiber can be added to the concentrated liquid. For example, unlimitatively, the gravitational acceleration (G) of centrifugation is 100G to 400G, and the size of the pore of the filter used for the filtration treatment is 10 µm to 100 µm. In addition, using magnetic fine particles coated, on the surface, with an antibody that specifically binds to the object cell, the cultured cells can be separated by magnetic force. Examples of such magnetic fine particles include Dynabeads (manufactured by Veritas Ltd.), MACS microbead (manufactured by Miltenyi Biotec), BioMag (manufactured by Techno Chemicals Corporation), and the like. Exchange of the medium composition can also be performed by using a bioreactor and an automatic incubator capable of conducting under a mechanical control and under a closed environment.

### Examples

The present invention is explained in more detail in the following Examples. The materials, amount of use, proportion, treatment content and treatment procedure shown in the following examples can be appropriately within the scope of the claims. Therefore, the scope of the present invention should not be construed as specific examples shown below.

### (Experimental Example 1: HEK293 cell proliferation and shake effect in 3D culture using chitin nanofiber)

Chitin nanofiber (biomass nanofiber BiNFi-S (BiNFi-s) 2 mass %, SUGINO MACHINE LIMITED) prepared according to the method described in WO 2015/111686 A1 was suspended in ultrapure water (Milli-Q water) to 1% (w/v), dissolved by stirring with heating at 90°C, and this aqueous solution was sterilized at 121°C for 20 min in an autoclave. A medium composition containing serum-free medium SFM Transfx293 medium (manufactured by HyClones) and 0.1% chitin nanofiber at a final concentration of 0.01% (w/v), and a medium composition without addition of the above-mentioned substrate were prepared. Successively, cultured human embryonic kidney cell line HEK293 (manufactured by DS Pharma Biomedical Co., Ltd.) was seeded in the above-mentioned medium composition added with chitin nanofiber to 133333 cells/mL, and dispensed to 15 mL in a 50 mL mini bioreactor (manufactured by Corning Incorporated, 431720). Each reactor was continuously cultured in a CO₂ incubator (37°C, 5% CO₂) in a state of standing or shaking (cfg 100 rpm) for 10 days. The culture medium on day 0 and day 10 was suspended by pipetting, ATP reagent (100 µL) (CellTiter-Glo™ Luminescent Cell Viability Assay, manufactured by Promega) was added to the cell suspension (100 µL) and the mixture was reacted and stood for about 10 min at room temperature. The luminescence intensity (RLU value) was measured by FlexStation3 (manufactured by Molecular Devices), the luminescence value of the medium alone was subtracted and the number of viable cells was measured as the average value of 4 points.

As a result, when HEK293 cells were cultured in the medium composition added with chitin nanofiber in the 50 mL mini bioreactor, a cell proliferation enhancing action due to the chitin nanofiber addition was found. Furthermore, when cultured with shaking, the proliferation enhancing effect of chitin nanofiber was further improved. The RLU value (ATP measurement, luminescence intensity) of each culture is shown in Table 1.

**[Table 1]**

| | | day 0 | day 10 |
|---|---|---|---|
| standing conditions | no addition | 24554 | 46340 |
| standing conditions | chitin nanofiber 0.01% | 25091 | 100707 |
| standing conditions | chitin nanofiber 0.01% | 25525 | 86930 |
| shaking conditions | no addition | 24199 | 83362 |
| shaking conditions | chitin nanofiber 0.01% | 25606 | 241622 |
| shaking conditions | chitin nanofiber 0.1% | 24817 | 191088 |

### (Experimental Example 2: HEK293 cell proliferation action in 3D culture using chitin nanofiber)

Chitin nanofiber (biomass nanofiber BiNFi-S (BiNFi-s) 2 mass %, SUGINO MACHINE LIMITED) prepared according to the method described in WO 2015/111686 A1 was suspended in ultrapure water (Milli-Q water) to 1% (w/v), dissolved by stirring with heating at 90°C, and this aqueous solution was sterilized at 121°C for 20 min in an autoclave. A medium composition containing serum-free medium SFM Transfx293 medium (manufactured by HyClones) and chitin nanofiber at a final concentration of 0.01% (w/v), and a medium composition without addition of the above-mentioned substrate were prepared. Successively, cultured human embryonic kidney cell line HEK293 (manufactured by DS Pharma Biomedical Co., Ltd.) was seeded in the above-mentioned medium composition added with chitin nanofiber to 13333 cells/mL or 33333 cells/mL, and dispensed to 15 mL in a 50 mL mini bioreactor (manufactured by Corning Incorporated, 431720). Each reactor was continuously cultured in a CO₂ incubator (37°C, 5% CO₂) in a state of shaking (cfg 100 rpm) for 7 days and 14 days. The culture media on days 0, 7, 14 were suspended by pipetting, ATP reagent (100 µL) (CellTiter-Glo™ Luminescent Cell Viability Assay, manufactured by Promega) was added to the cell suspension (100 µL) and the mixture was reacted and stood for about 10 min at room temperature. The luminescence intensity (RLU value) was measured by FlexStation3 (manufactured by Molecular Devices), the luminescence value of the medium alone was subtracted and the number of viable cells was measured as the average value of 4 points.

As a result, when HEK293 cells were cultured in the medium composition added with chitin nanofiber in the 50 mL mini bioreactor, a cell proliferation enhancing action due to the chitin nanofiber addition was found even in low density culturing with a small number of seeding. The RLU value (ATP measurement, luminescence intensity) on day 7 of culture is shown in Table 2 and the RLU value (ATP measurement, luminescence intensity) on day 14 of culture is shown in Table 3.

**[Table 2]**

| number of seeded cells | | day 0 | day 7 |
|---|---|---|---|
| 13333 cells/mL | no addition | 3785 | 17555 |
| 13333 cells/mL | chitin nanofiber 0.01% | 3687 | 32166 |
| 33333 cells/mL | no addition | 8656 | 24006 |
| 33333 cells/mL | chitin nanofiber 0.01% | 8529 | 57849 |

**[Table 3]**

| number of seeded cells | | day 0 | day 14 |
|---|---|---|---|
| 13333 cells/mL | no addition | 3899 | 40442 |
| 13333 cells/mL | chitin nanofiber 0.01% | 3939 | 127986 |
| 33333 cells/mL | no addition | 8573 | 46483 |
| 33333 cells/mL | chitin nanofiber 0.01% | 8898 | 102441 |

### (Reference Example 1: Preparation of IFN-β-producing HEK293 cell)

Human interferon β (IFN-β) gene was incorporated into HEK293 as shown below and a cell line that produces IFN-β in the culture supernatant was produced.

First, IFN-β gene was obtained from human skin-derived normal diploid fibroblast cell line TIG-108, which is IFN-β-producing cell. TIG-108 (5×10⁶ cells) were centrifuged at 400×g for 5 min, the supernatant was removed, a nucleic acid extraction reagent ISOGEN (NIPPON GENE) (1 mL) and chloroform (Wako Pure Chemical Industries, Ltd.) (0.2 mL) were added, and DNA and protein were precipitated in the organic layer. After centrifugation at 12000×g for 15 min, the aqueous phase (RNA layer) was obtained, isopropanol (Wako Pure Chemical Industries, Ltd.) (0.5 mL) was added thereto, and the mixture was centrifuged at 12000xg for 15 min and RNA was precipitated. The precipitated RNA was dissolved in sterile water, First-Stand Reaction Mix Beads (Amersham Bioscience) and pd(N)6 Randam Hexamer (Amersham Bioscience) 1 µL were added, and the mixture was stood at room temperature for 1 min. After standing, reverse transcription reaction was performed at 37°C for 60 min to synthesize cDNA.

Successively, PCR amplification of IFN-β gene was tried. First, sterile water (35.5 pL), Blend Taq buffer (5 µL), dNTP mix (4 pL), Primer F (5'-ATGACCAACAAGTGTCTCCT-3'; SEQ ID NO: 1) (1 pL) (10 pmoles), Primer R (5'-TCAGTTTCGGAGGTAACCTG-3'; SEQ ID NO: 2) 1 µL (10 pmoles), Taq DNA polymerase (0.25 µL) (Takara Bio) and cDNA (3 µL) (50 ng) were mixed in a 0.2 mL tube. This tube was placed in Program Temp. Control System (ASTEC) and PCR was conducted. PCR product was confirmed by 1% agarose gel electrophoresis and purified using Freeze'N Squeese spin column (BIO RAD). The purified PCR product (2 µL) and pTARGET vector (1 µL) (50 ng) were mixed with TaKaRa DNA Ligation Kit (Takara Bio) (3 µL) and a ligation reaction was performed at 16°C for 30 min. Successively, pTARGET vector after ligation was introduced into JM109 cells (Promega) by a heat shock reaction, and the cells were seeded in an LB medium plate. The grown colonies were obtained, cultured with shaking in LB medium (1 mL) at 37°C overnight, and pTARGET vector was extracted with QIAprep Spin Miniprep Kit (QIAGEN). Successively, the pTARGET vector was cleaved with a restriction enzyme PstI (TOYOBO) having a recognition sequence on the IFN-β gene sequence, whereby it was confirmed that the IFN-β gene was cloned into the pTARGET vector. Successively, HEK293 cell line was cultured in 10% fetal bovine serum (FBS, Trace)-containing -E-RDF (10% FBS-E-RDF) medium, adjusted to 5×10⁶ cells/mL and replaced with PBS. Then, it was confirmed that the IFN-β gene was cloned into the pTARGET vector.

The pTARGET vector (3 pg) incorporating the IFN-β gene was added, and voltage was applied under the conditions of 0.75kV/cm, 350 µF. Recovery culture was carried out in 10% FBS-E-RDF medium, and the cells were dispensed to a 96 well culture plate (Becton & Dickinson) at 100 µL/well. After incubation for 24 hr, transfected cells were selectively cultured in 10% FBS-E-RDF medium added with G-418 (Geneticin, Invitrogen) to a final concentration of 500 pg/mL. Thereafter, the cells confirmed to have proliferated were confirmed for IFN-β gene expression, whereby IFN-β-producing HEK293 cells were produced.

### (Experimental Example 3: Proliferation of IFN-β-producing HEK293 cells and IFN-β secretion in 3D culture using chitin nanofiber)

Chitin nanofiber (biomass nanofiber BiNFi-S (BiNFi-s) 2 mass %, SUGINO MACHINE LIMITED) prepared according to the method described in WO 2015/111686 A1 was suspended in ultrapure water (Milli-Q water) to 1% (w/v), dissolved by stirring with heating at 90°C, and this aqueous solution was sterilized at 121°C for 20 min in an autoclave. A medium composition containing serum-free CD293 medium for suspension culture (manufactured by Thermofisher Scientific) and chitin nanofiber at a final concentration of 0.003%, 0.01%, 0.03% (w/v), and a medium composition without addition of the above-mentioned substrate were prepared. Successively, cultured IFN-β-producing HEK293 cells were seeded in the above-mentioned medium composition added with chitin nanofiber to 133333 cells/mL, and dispensed to 15 mL in a 50 mL mini bioreactor (manufactured by Corning Incorporated, 431720). Each reactor was continuously cultured in a CO₂ incubator (37°C, 5% CO₂) in a state of shaking (cfg 100 rpm) for 7 days and 14 days. The culture media on days 0, 7, 14 were suspended by pipetting, ATP reagent (100 µL) (CellTiter-Glo™ Luminescent Cell Viability Assay, manufactured by Promega) was added to the cell suspension (100 µL) and the mixture was reacted and stood for about 10 min at room temperature. The luminescence intensity (RLU value) was measured by FlexStation3 (manufactured by Molecular Devices), the luminescence value of the medium alone was subtracted and the number of viable cells was measured as the average value of 4 points. In addition, the cell culture media on days 7, 14 were centrifuged (200G, 3 min), and the culture supernatant was dispensed.

### [Measurement of IFN-β production amount by enzyme antibody method]

The amount of IFN-β produced in of the culture supernatant was measured by an enzyme antibody method (ELISA; enzyme-linked immunosorbent assay). Goat anti-human IFN-β antibody (R&D System INC.) was diluted with phosphate buffered saline (PBS) to 1 µg/mL and dispensed to a 96 well immuno plate at 100 µL/well to coat the plate. After standing at room temperature for 1 hr, to prevent non-specific reactions, bovine serum albumin (BSA; ICN) was diluted with PBS to 1% and the solution (1% BSA/PBS) was dispensed at 150 µL/well to perform blocking. After standing at room temperature for 1 hr, the culture supernatant containing IFN-β antibody to be quantified was dispensed at 50 µL/well. Similarly, a standard solution (0 µg/mL-200 µg/mL) was dispensed at 50 µL/well, and the mixture was stood at room temperature for 1 hr. Thereafter, mouse anti-human IFN-β antibody (R&D System INC.) was diluted with 1% BSA/PBS to 1 µg/mL, dispensed at 100 µL/well, and stood at room temperature for 1 hr. Thereafter, horseradish peroxidase-labeled rat anti-mouse IgG antibody was diluted about 6,000-fold with 1% BSA/PBS solution, dispensed at 100 µL/well and stood at room temperature for 1 hr. As a color developing solution, a 10% 3,3',5,5'-tetramethylbenzidine (TMB) color developing solution (manufactured by Funakoshi Co., Ltd., #TMBW-100-0) was dispensed at 100 µL/well, the color development was confirmed and the reaction was discontinued with STOP Solution (manufactured by KPL, #50-85-04), and the absorbance was measured at 450 nm wavelength. Between respective reactions, the plate was washed 3 times with a solution of polyethylene (20) sorbitan monolaurate (Tween 20) (Wako Pure Chemical Industries, Ltd.) diluted with PBS to a concentration of 0.05%.

As a result, when IFN-β-producing HEK293 cells were cultured in the medium composition containing chitin nanofiber in the 50 mL mini bioreactor, a cell proliferation enhancing action due to the chitin nanofiber addition was found. In addition, the amount of human IFN-β in the culture supernatant increased. From the results, it was clarified that culturing with shaking in the medium composition containing chitin nanofiber promotes both cell proliferation and IFN-β production. The RLU value (ATP measurement, luminescence intensity) on day 7 of culture is shown in Table 4, the RLU value (ATP measurement, luminescence intensity) on day 14 of culture is shown in Table 5, and human IFN-β secretion amount (ng/mL) is shown in Table 6.

**[Table 4]**

| | day 0 | day 7 |
|---|---|---|
| no addition | 18001 | 22193 |
| chitin nanofiber 0.003% | 18202 | 16084 |
| chitin nanofiber 0.01% | 18919 | 46861 |
| chitin nanofiber 0.03% | 19572 | 63608 |

**[Table 5]**

| | day 0 | day 14 |
|---|---|---|
| no addition | 19435 | 26340 |
| chitin nanofiber 0.003% | 18843 | 58300 |
| chitin nanofiber 0.01% | 18465 | 47495 |
| chitin nanofiber 0.03% | 18512 | 55070 |

**[Table 6]**

| | day 7 | day 14 |
|---|---|---|
| no addition | 4.83 | 6.64 |
| chitin nanofiber 0.003% | 3.77 | 9.39 |
| chitin nanofiber 0.01% | 7.06 | 12.79 |
| chitin nanofiber 0.03% | 6.82 | 19.88 |

### (Experimental Example 4: MDCK cell proliferation and shake effect in 3D culture using chitin nanofiber)

Chitin nanofiber (biomass nanofiber BiNFi-S (BiNFi-s) 2 mass %, SUGINO MACHINE LIMITED) prepared according to the method described in WO 2015/111686 A1 was suspended in ultrapure water (Milli-Q water) to 1% (w/v), dissolved by stirring with heating at 90°C, and this aqueous solution was sterilized at 121°C for 20 min in an autoclave. A medium composition containing serum-free medium SFM Transfx293 medium (manufactured by HyClones) and 0.1% chitin nanofiber at a final concentration of 0.01% (w/v), and a medium composition without addition of the above-mentioned substrate were prepared. Successively, cultured canine kidney renal tubule epithelial cell line MDCK (manufactured by DS Pharma Biomedical Co., Ltd.) was seeded in the above-mentioned medium composition added with chitin nanofiber to 133333 cells/mL, and dispensed to 15 mL in a 50 mL mini bioreactor (manufactured by Corning Incorporated, 431720). Each reactor was continuously cultured in a CO₂ incubator (37°C, 5% CO₂) in a state of standing or shaking (cfg 100 rpm) for 10 days. The culture media on day 0 and day 10 were suspended by pipetting, ATP reagent (100 µL) (CellTiter-Glo™ Luminescent Cell Viability Assay, manufactured by Promega) was added to the cell suspension (100 µL) and the mixture was reacted and stood for about 10 min at room temperature. The luminescence intensity (RLU value) was measured by FlexStation3 (manufactured by Molecular Devices), the luminescence value of the medium alone was subtracted and the number of viable cells was measured as the average value of 4 points.

As a result, when MDCK cells were cultured in the medium composition containing chitin nanofiber in the 50 mL mini bioreactor, a cell proliferation enhancing action due to the chitin nanofiber addition was found. Furthermore, when cultured with shaking, the cell proliferation enhancing effect of chitin nanofiber was further improved. The RLU value (ATP measurement, luminescence intensity) of each culture is shown in Table 7.

**[Table 7]**

| | | day 0 | day 10 |
|---|---|---|---|
| standing conditions | no addition | 11707 | 12708 |
| standing conditions | chitin nanofiber 0.01% | 12084 | 58282 |
| standing conditions | chitin nanofiber 0.01% | 12655 | 73354 |
| shaking conditions | no addition | 12427 | 3763 |
| shaking conditions | chitin nanofiber 0.01% | 12263 | 155610 |
| shaking conditions | chitin nanofiber 0.1% | 12013 | 112137 |

### [Industrial Applicability]

According to the present invention, it is possible to cause efficient proliferation of the cells, and produce proteins such as enzyme, cell growth factor, antibody and the like on a large scale.

## Claims

1. A method for producing a protein, comprising culturing a cell having protein production ability in suspension in a medium composition comprising nanofibers under physical disruption condition and in a state of being attached to the nanofibers, wherein the nanofibers are constituted of a non-water-soluble polysaccharide selected from the group consisting of cellulose, chitin and chitosan,
the cell is HEK293, CHO-K1, BHK-21, MDCK, Vero, HepG2 or MCF-7, and
the nanofibers in the medium composition are present at a concentration within a range that can promote the protein production and/or proliferation by the cell to be cultured.

2. The method according to claim 1, wherein the nanofibers are chitin nanofibers.

3. The method according to claim 2, wherein the content of the chitin nanofibers in the medium composition is 0.003 - 0.1% (weight/volume).

4. The method according to any one of claims 1 to 3, wherein the cell is an adherent cell.

## Patentansprüche

1. Verfahren zur Herstellung eines Proteins, umfassend das Kultivieren einer Zelle, die zur Proteinproduktion befähigt ist, in Suspension in einer Mediumzusammensetzung, die Nanofasern umfasst, unter physikalischen Lysebedingungen und in einem Zustand des Gebundenseins an die Nanofasern, wobei die Nanofasern aus einem nichtwasserlöslichen Polysaccharid aufgebaut sind, das aus der Gruppe ausgewählt ist, die aus Cellulose, Chitin und Chitosan besteht, es sich bei der Zelle um HEK293, CHO-K1, BHK-21, MDCK, Vero, HepG2 oder MCF-7 handelt und die Nanofasern in der Mediumzusammensetzung in einer Konzentration innerhalb eines Bereichs vorhanden sind, die die Proteinproduktion und/oder -vermehrung durch die zu kultivierende Zelle fördern kann.

2. Verfahren gemäß Anspruch 1, wobei die Nanofasern Chitin-Nanofasern sind.

3. Verfahren gemäß Anspruch 2, wobei der Gehalt an Chitin-Nanofasern in der Mediumzusammensetzung 0,003 bis 0,1% (w/v) beträgt.

4. Verfahren gemäß einem der Ansprüche 1 bis 3, wobei die Zelle eine adhärente Zelle ist.

## Revendications

1. Méthode de production d'une protéine, comprenant la mise en culture d'une cellule ayant une capacité de production de protéine en suspension dans une composition de milieu comprenant des nanofibres dans des conditions de perturbation physique et dans un état de fixation aux nanofibres,
dans laquelle les nanofibres sont constituées d'un polysaccharide non soluble dans l'eau sélectionné dans le groupe constitué par la cellulose, la chitine et le chitosane,
la cellule est HEK293, CHO-K1, BHK-21, MDCK, Vero, HepG2 ou MCF-7, et
les nanofibres dans la composition de milieu sont présentes à une concentration située dans une plage qui peut favoriser la production de protéine et/ou la prolifération par la cellule à cultiver.

2. Méthode selon la revendication 1, dans laquelle les nanofibres sont des nanofibres de chitine.

3. Méthode selon la revendication 2, dans laquelle la teneur en nanofibres de chitine dans la composition de milieu est 0,003 -0,1 % (en poids/volume).

4. Méthode selon l'une quelconque des revendications 1 à 3, dans laquelle la cellule est une cellule adhérente.
